# EUROPEAN PATENT APPLICATION

(11) **EP 1 970 671 A1**
(43) Date of publication of application: **17.09.2008**
(21) Application number: 08380038.3
(22) Date of filing: 13.02.2008
(51) Int. Cl.: G01C 22/00, A43B 5/06, A61B 5/103

(54) **Intelligent shoe**

(30) Priority: 16.03.2007 ES 200700705
(71) Applicant: Calzados Hergar, S.A., E-26580 Arnedo, (La Rioja) (ES)
(72) Inventor: García Moron, Basilio, 26580 Arnedo (La Rioja) (ES)
(74) Representative: Villamor Muguerza, Jon

(57) **Abstract**

The invention relates to an intelligent shoe especially designed for its user to have a control of different variables which are presented when walking, such as the distance traveled, the time used, the calories consumed, etc, the shoe is formed from an upper (1) and a sole (2), suitable with any line of design, a sole in which a practicable package (4) is provided, internally provided with an electronic circuit (5), in which a step detector (6) is provided according to the time and an emitter (7), such that the mentioned shoe is complemented with a receiver device (8) wirelessly associated to the emitter (7), provided with fixing/hanging means, a receiver device (8) provided with an internal memory for storing the data captured by the circuit (5), as well as at least one connection port (9) to a computer (10) for dumping and processing the data by means of a computer application which allows establishing a personal training program for the user.

## Description

### Object of the Invention

The present invention relates to a shoe which has been specifically conceived to allow its user to have control over different variables presented when walking, such as the distance traveled, the time used, the calories consumed, etc.

The object of the invention is to provide a device with reduced electric consumption, small size and weight, which can be easily removed from the shoe to which it is coupled, allowing the data related to the user to be stored in order to be able to subsequently dump the data in a computer or the like, which through a certain software allows providing a personal training program for the user, advising him or her on guidelines based on the selected objective, such as losing weight, maintaining physical fitness, post-heart attack recovery, reducing triglycerides, etc.

### Background of the Invention

As is known the habit of walking is an exercise from which a number of benefits can be derived if it is practiced in a continuous and controlled manner. Among other benefits which are derived from this practice the following are emphasized:
- Preventing cardiovascular diseases from occurring.
- Recovering after a heart attack.
- Increasing HDL cholesterol (protector). Decreasing triglycerides.
- Decreasing the blood pressure at rest.
- Decreasing and controlling excess weight.
- Increasing bone density (preventing osteoporosis).
- Maintaining physical fitness by means of increasing aerobic capacity.

However the great problem of practicing any physical exercise is abandoning said activity due to the inability of making it a habit, with the necessary continuity and persistence by the individual.

In this sense the solution is to create a faithful link with the individual, for the purpose of motivating him or her to adopt a healthy habit and not to abandon it.

In the field of shoes, a number of devices related to the concept of an "active shoe" are known such as means for monitoring steps, pressure, load and other variables taking part in the activity of walking, as well as shoes provided with means of lighting, vibration and audio, temperature regulation, soles formed by temperatures, etc, the huge majority of which have not been as well received in the market as expected, their success being rather limited.

The applicant has no knowledge of the existence of a shoe which allows motivating its user to continuously and regularly exercise, specifically the habit of walking, which allows following an evolution over time, both short term and long term of the physical fitness thereof.

### Description of the Invention

The device for the shoe proposed by the invention fully and satisfactorily solves the drawbacks previously set forth, providing a shoe allowing its user to carry out a continuous follow-up of his or her physical activity related to the act of walking, allowing a follow-up of the evolution of the data obtained when walking to be carried out, resulting in a dynamically adaptive interactive device according to, the profile of the user, which allows establishing goals/challenges for the user with a personalized training program.

To that end the proposed shoe starts from the basic structure of any conventional shoe, with the special particularity that in its sole a practicable package is incorporated, which is perfectly integrated in the aesthetics of said sole in the coupling arrangement, a package in which an electric circuit basically based on an electronic circuit provided with a step detector with regard to time is provided, said circuit being wirelessly associated with a small-sized receiver device, which can be established in the belt of the user, integrated in a bracelet, in a necklace or simply in one of the pockets of the user, which collects the data generated while exercising and stores it for later analysis.

Said receiver device incorporates means for connecting to a computer in order to download the mentioned information, the invention being complemented with a personal training software for the user, although it can also incorporate a small LED display showing the number of steps taken by the user or an LED bar as a economical alternative and optionally substituting said display.

The data collected during the use of the shoe are processed by the mentioned programming software in order to calculate a series of parameters which allow informing the user about the physical activity being carried out and advising him or her on guidelines based on the selected objective (losing weight, maintaining physical fitness, post-heart attack recovery, reducing triglycerides, decreasing blood pressure at rest, etc.).

The mentioned application can work both through a web environment as well as without the need of an Internet connection in the computer in which it is installed, all this with an attractive design, dynamic adaptation to the profile of the user and allowing him or her to establish goals/challenges.

A device is thus achieved which allows establishing the habit of walking in a simple and attractive manner, from which a number of benefits can be derived for the health of the user.

### Description of the Drawings

To complement the description being made and for the purpose of aiding to better understand the features of the invention according to a preferred practical embodiment thereof, a single page of drawings is attached as an integral part of said description, in which the different elements taking part in the intelligent shoe of the invention have been schematically depicted with an illustrative and non-limiting character.

### Preferred Embodiment of the Invention

In view of the indicated figure it can be observed how the proposed shoe is formed from an upper (1), and a sole (2), both suitable for any line of design, with the special particularity that the mentioned sole (2) includes a housing (3) in which a preferably practicable package (4) is provided, which package contains an electronic circuit (5), with very low electric consumption, provided with a step detector (6) and an emitter (7), such that said circuit (5) wirelessly sends the information relating to the number of steps according to the time passed to a receiver device (8) responsible for storing said information, and which will be discussed further on.

The mentioned electronic circuit (5) will be supplied by the corresponding battery or batteries, not shown in the drawings, having been provided that the autonomy thereof is not less than 1 year, having a compact architecture, a very small size, being integrally housed within the package (4) which will lack outer electronic elements.

The practicable feature of said package (4) allows the shoe to be used in airport checkpoints and the like, such that by means of removing said package from the sole (2) of the shoe, it can be used as a conventional shoe with no problem when passing through said checkpoints. For such purpose it has been provided that the shoe has ; a second package with similar outer features to the package (4) but without inner electronic devices, in order to allow sealing the housing which is visible upon uncoupling said package (4).

The step detector (6) can be base on five different technologies without affecting the essential nature of the invention. In this sense it can be formed from infrared sensor and emitter, ultrasonic sensor and emitter, piezoelectric sensor, push-button reed switch or resistive ink sensor technologies, whereas the wireless communication (7) is carried out in a miniature transmission module in the 2.4 GHz band.

The position of the housing (3) will depend on the type of sensor of those previously described, such that it can be located indistinctly in the heel or waist area.

Returning to the receiver device (8), this will be integrated within a protective casing, corresponding with reference number (8), preferably with a parallelepiped geometry, of a small size, low weight, with the possibility of being fixed to the belt, waist of the pants, also being able to be carried in a pocket or having hanging means.

Said receiver device (8) gathers the data generated during exercising and keeps it stored in an internal memory to be downloaded through a connection port (9) to a PC (10), preferably a USB port.

The receiver device (8) can optionally have a screen or display (11) through which the number of steps taken by the user is shown, or it can incorporate an LED bar.

The data collected during the use of the shoe and once it is dumped into the computer (10) are processed by means of a computer application by way of a personal trainer, designed to be used in a web environment, either on-line or off-line.

Said application is responsible for calculating a multitude of parameters from the two variables which the device collects, the number of steps per unit of time, such as the distance traveled, calories burned, type of exercise and quality thereof, etc, informing the user about the physical activity carried out and advising him or her on guidelines based on the selected objective from among a series of options (loosing weight, maintaining physical fitness, post-heart attack recovery, reducing triglycerides, etc).

The application will preferably have an attractive graphical design, with the use of flash technologies in order to provided dynamism to the page, dynamically adapting itself according to the profile of the user such that the application has the appearance of a virtual entity in its interaction with the user, it being in charge of making said user loyal to his or her physical activity routine, creating good feelings based on close and personal communication. Said application must also establish goals/challenges for the user, allowing a follow-up of the evolution of the data to be carried out, storing series over the short term and long term for the purpose of being able to compare the results and making interaction with other users possible.

Said application can be linked to a web maintenance service stored in a conventional server.

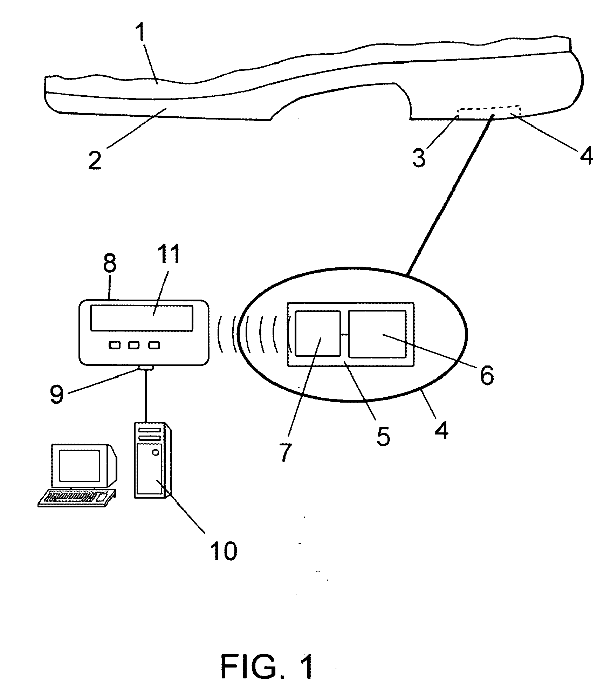

## Claims

1. An intelligent shoe, of the type of those formed from an upper (1) and a sole (2), suitable with any line of design, **characterized in that** within the sole (2) an electronic circuit (5) is provided, of low electric consumption, provided with a step detector (6) according to the time and an emitter (7), having been provided that the mentioned shoe is complemented with a receiver device (8) wirelessly associated to the emitter (7), provided with an internal memory for storing the data captured by the circuit (5), as well as at least one connection port (9) to a computer (10) for dumping and processing the data by means of a computer application.

2. An intelligent shoe according to claim 1, **characterized in that** the mentioned electronic circuit (5) is provided within a practicable package (4), able to be coupled in a housing (3) suitable in shape and size provided in the sole (2) of the shoe, having been provided that said shoe has a second package with no electronic devices, of similar outer features to the package (4), for the substitution thereof.

3. An intelligent shoe according to claim 1, **characterized in that** the step detectors (6) are indistinctly based on infrared sensor and emitter, ultrasonic sensor and emitter, piezoelectric sensor, push-button reed switch or resistive ink sensor technologies. ,

4. An intelligent shoe according to claim 1, **characterized in that** the means for wireless communication between circuits (5-8) are carried out in a miniature transmission module in the 2.4 GHz band.

5. An intelligent shoe according to the previous claims, **characterized in that** the receiver device (8) optionally has a screen or display (11) for displaying the information collected, or in its absence an LED bar.

6. An intelligent shoe according to claim 1, **characterized in that** the receiver device (8) is integrated in a small-sized casing provided with fixing/stabilizing and/or hanging means.
